Europäisches Patentamt

**European Patent Office**

**Office européen des brevets**

(11) Publication number: **0 173 293 B1**

# EUROPEAN PATENT SPECIFICATION

(45) Date of publication of patent specification: **30.10.91**   (51) Int. Cl.⁵: **A61K 31/445, A61K 9/66**

(21) Application number: **85110726.8**

(22) Date of filing: **27.08.85**

The file contains technical information submitted after the application was filed and not included in this specification

(54) **Hot melt antihistamine formulations.**

(30) Priority: **30.08.84 US 646179**

(43) Date of publication of application:
**05.03.86 Bulletin 86/10**

(45) Publication of the grant of the patent:
**30.10.91 Bulletin 91/44**

(84) Designated Contracting States:
**AT BE CH DE FR GB IT LI LU NL SE**

(56) References cited:
**EP-A- 0 086 468**
**EP-A- 0 107 085**
**US-A- 3 139 383**
**US-A- 3 878 217**

(73) Proprietor: **MERRELL DOW PHARMACEUT-ICALS INC.**
**2110 East Galbraith Road**
**Cincinnati Ohio 45215-6300(US)**

(72) Inventor: **Withington, Roger**
**58 Abbots Ride**
**Farnham Surrey GU9 8HZ(GB)**

(74) Representative: **Sgarbi, Renato et al**
**GRUPPO LEPETIT S.p.A. Patent and Trade-mark Department 34, Via Roberto Lepetit**
**I-21040 Gerenzano (Varese)(IT)**

## Description

Background of the Invention

Terfenadine, α-(p-tert-butylphenyl)-4-(α-hydroxy-α-phenylbenzyl)-1-piperidinebutanol, is a known antihistamine and antiallergy agent. This compound and its properties are fully described in U.S.-A-3,878,217 and is commercially available in tablet form.

It would be highly desirable to provide a terfenadine formulation that would be suitable for use with either hard or soft shelled gelatin capsules. Not only do some patients have a preference for taking a drug in capsule form, but capsule formulations generally permit a drug to be administered in a more concentrated manner and with fewer excipients added. Moreover, capsules are tasteless, easily administered and capsule formulations are free of various additives such as lubricants, binders, dispersing agents, coatings and flavoring agents usually required with tablet formulations.

Both hard and soft gelatin capsules can be filled with liquid, semi-liquid, paste or solid formulations. The development of the rotary die process for the manufacture of soft gelatin capsules some 50 years ago has led, in general, to the choice of soft gelatin capsules for liquid or semi-liquid formulations, and to hard capsules for dry or granular formulations.

However, hard gelatin capsules that are liquid filled have a tendancy to leak. There is always a small air space between the cap and the body which permits products of low viscosity or low surface tension to leak by capillarity. In order to avoid leaking and to protect the contents of the capsule, hard shell capsules can be hermetically sealed by wetting the edges where the cap and body of the capsule overlap. Alternatively, capsules can be sealed by banding or the edges fused by heating in order to prevent leakage.

More recently there has been a renewed interest in the use of hot melt liquid or paste formulations. Such compositions are utilized while hot to fill the capsule body. Upon cooling these compositions set up as a solid fill that is stable and will not leak. Alternatively, at ambient temperatures, thixotropic formulations can be employed. Thixotropic compositions behave as liquids and remain fluid while under shear conditions. While in this state capsules are readily liquid filled. Upon standing, however, these formulations quickly set up and become a semi-solid or gel-like substance in the capsule, thereby preventing subsequent leakage.

ED-A-107085 describes use of anhydrous emulsions consisting of an oil phase and a phase of water-soluble, but anhydrous liquid, for filling gelatin capsules.

The present invention relates to hot melt terfenadine formulations that are suitable for use with either hard or soft gelatin capsules. These formulations are fluid while hot, thereby readily permitting capsule bodies to be liquid filled. Upon cooling, however, these formulations set up as a solid mass within the capsule that is highly stable and will not leak. Moreover, these formulations are easily prepared, can be readily stored in bulk form, and provide unique and heretofore unavailable sustained release characteristics for terfenadine.

Summary of the Invention

This invention relates to hot melt terfenadine compositions that are useful for the hot melt filling of hard or soft shell capsules. More particularly, the compositions of this invention comprise from 8 to 28 parts by weight of terfenadine or a pharmaceutically acceptable salt thereof, from 1 to 5 parts by weight of paraffin wax, and from 68 to 92 parts by weight of polyethylene glycol. Still more particularly, the compositions of this invention relate to dosage unit forms of terfenadine which contain from 60 to 180 mg of terfenadine or a pharmaceutically acceptable salt thereof, from 1 to 33 mg of paraffin wax and from 450 to 610 mg of polyethylene glycol.

Detailed Description of the Invention

In accordance with the present invention there are provided certain hot melt compositions of terfenadine for use in filling either two-piece hard or soft shell capsules. The term hot melt refers to a formulation having thermosoftening properties which permits it to melt and flow as a liquid at elevated temperatures, without any thermal degradation or decomposition of terfenadine. Upon cooling at temperatures below 60° C the compositions of this invention rapidly solidify and remain in a solid state unless reheated again at temperatures above 60° C.

In general, the compositions of this invention comprise three distinct components: terfenadine or a pharmaceutically acceptable salt thereof, a hydrophobic substance such as paraffin wax, and a hydrophilic substance such as polyethylene glycol. In order to obtain the proper thermosoftening and thermosetting

EP 0 173 293 B1

characteristics for the hot melt compositions described herein, while at the same time providing for the desired sustained release characteristics of terfenadine, the amounts, choice and the proportions of the particular hydrophobic and hydrophilic components employed are deemed to be critical.

The term "pharmaceutically acceptable salt" is intended to include suitable inorganic or organic acid addition salts of terfenadine and its optical isomers, which are both non-toxic and pharmacologically acceptable in humans. Suitable inorganic acids, include hydrochloric, hydrobromic, sulfuric, and phosphoric acids. Suitable organic acids, include carboxylic acids such as acetic, propionic, glycolic, lactic, pyruvic, malonic, succinic, fumaric, malic, tartaric, citric, ascorbic, maleic, hydroxymaleic, and dihydroxymaleic: aromatic acids such as benzoic. phenylacetic. 4-aminobenzoic, 4-hydroxy-benzoic, anthranilic, cinnamic, salicylic, 4-aminosalicylic, 2-phenoxybenzoic, 2-acetoxybenzoic, and mandelic acids; and sulfonic acids, such as methanesulfonic, ethanesulfonic and $\beta$-hydroxyethanesulfonic acids. The amount of terfenadine base employed in the compositions of this invention ranges from 8 to 28 parts by weight of the total composition, or from 60 mg to 180 mg per unit dose. Preferably, 15 to 20 parts by weight and 100 to 140 mg of terfenadine per unit dose are employed. Obviously, if a salt is employed, the molecular weight of the particular salt moiety must be taken into consideration.

The hydrophobic substance must essentially satisfy two specific requirements. First, it must inhibit or slow down the release of terfenadine from the formulation, thereby enabling a patient to obtain allergenic relief over a reasonable period of time. Secondly, the hydrophobic substance must be compatible with both terfenadine and the hydrophilic component utilized, while at the same time not appreciably altering the desired hot melt characteristics of the finished composition.

Various hydrophobic substances have been utilized. However, pharmaceutical grade paraffin wax, having a m.p. of 52-56°C, has been found to be particularly useful in the practice of this invention. Depending upon the particular hydrophilic component employed, the amount of paraffin wax employed ranges anywhere from 1 to 5 parts by weight of the total composition, or from 1 to 33 mg per dosage unit. Preferably, 1 part by weight or from 5 to 10 mg per dosage unit of paraffin wax is employed.

The hydrophilic component essentially provides a solid matrix from which the dissolution of terfenadine occurs once the gelatin capsule has been ruptured. The choice of this vehicle is deemed to be critical. If the hydrophilic component is too low melting and/or is too water soluble, terfenadine will be too rapidly released into the blood stream, resulting in a short acting dosage unit form. Thus, a patient would require the administration of multiple doses in order to obtain any reasonable period of effective relief.

On the other hand, if the hydrophilic substance melts too high and/or is too water soluble, the absorption of terfenadine will be unreasonably delayed thereby providing little, if any, effective relief for the patient. In addition, the thermo-softening properties of the hydrophilic substance must be of such a nature as to permit the composition to melt and flow at elevated temperatures which do not result in a thermal degradation or decomposition of terfenadine. Finally, the hydrophilic substance must have thermosetting characteristics which enable the finished formulation to re-solidify and remain solid at temperatures below 60°C.

In this regard, polyethylene glycols have been found to be particularly useful in satisfying all of these requirements for the hydrophilic component. More particularly, polyethylene glycols having an average molecular weight ranging from about 2000 to about 6000 are especially useful in the practice of this invention. Still more particularly, polyethylene glycol having an average molecular of about 3350 is the preferred hydrophilic component to be employed in the practice of this invention.

Depending upon the particular equipment utilized for the hot melt filling of capsules, it may be desirable to employ a composition that is thixotropic in nature. The term thixotropic as used herein refers to that property of a composition which causes it to behave as a free flowing liquid under shear conditions, as for example, with stirring or in pumping. However, upon subsequent standing such compositions quickly set up to become semi-solid or gel-like. The use of agents, such as finely divided silicon dioxide (Aerosil®), beeswax or small amounts of high molecular weight polyethylene glycol, serves to impart thixotropic properties to the compositions of the present invention.

One of the principal advantages of this invention is to provide a convenient dosage unit form of terfenadine that needs to be administered only once each day. At present terfenadine must be administered in tablet form two or three times daily to patients in need thereof in order to provide effective relief. It would be highly desirable to be able to provide such patients with an easily administered capsule form of terfenadine that needs to be taken only once every 24 hours.

The hot melt compositions described herein are particularly useful for this purpose in that they provide a steady and sustained dissolution of terfenadine over a prolonged period of time. Thus, patients taking capsules containing these compositions require only one such capsule per day in order to obtain quick, effective and long-lasting relief, a result which heretofore was unavailable.

3

A further advantage of the present invention is that the hot melt compositions described herein can be easily and readily prepared. In general, these compositions are prepared by first melting the hydrophilic substance, polyethylene glycol, at a temperature from about 60 to 80° C. Preferably, a temperature of 70° C is employed. The active ingredient, terfenadine, and the hydrophobic substance, in this instance paraffin wax, is added to the polyethylene glycol melt with constant mechanical stirring. At this point, the composition can either be directly introduced into a capsule filling machine and utilized without further preparation, or it can be homogenized for approximately 30 minutes at 70° C in order to obtain a more uniformly dispersed hot melt composition. If the hot melt composition is not to be immediately utilized, it can be poured onto trays and cooled for subsequent utilization. The cooled product can be stored as such or broken, screened and subsequently employed as a liquid fill in capsule fillers equipped with heated hoppers.

Still another advantage of the present invention is that no particular or special equipment is required to fill commercially available capsule blanks. Any commercially available capsule shell filling machine equipped for hot melt filling can be employed. In general, equipment of this type is provided with thermostatically controlled heated hoppers, metering pumps and nozzles to keep the compositions fluid until the capsules are filled. Once filled, the capsules are rapidly cooled in order to solidify the fill as quickly as possible.

Thus, it can be seen that the present formulations are readily and simply prepared. They do not require special ingredients such as lubricants, binders, disintegrants or flow aids. No dust is generated in the filling of capsules. Furthermore, more accurate dosage fills can be obtained utilizing present day automatic liquid filled capsule filling machines in lieu of conventional granular capsule filling equipment.

The following examples are provided to further illustrate the invention.

EXAMPLE 1

### Ingredients

| | | |
|---|---|---|
| Terfenadine | 0.60 | kg |
| Polyethylene glycol 3350 USP | 2.72 | kg |
| Paraffin wax (m.p. 52-6°C) | 0.034 | kg |

The polyethylene glycol is melted in a suitable container and maintained at 70° C. Terfenadine and the paraffin wax are added to the hot melt with constant mechanical stirring. The resulting mixture is homogenized for 30 minutes at 70° C, poured onto trays and allowed to cool. The cooled product is broken into pieces and passed through an oscillating granulator using a 1.6 mm screen. The resulting composition can be utilized in combination with any standard hot melt, hard or soft shell capsule filling machine such as Hofliger and Karg, GKF 120L.

EXAMPLE 2

Using the composition of Example 1, an injection fill of 670 mg into a standard Size 0 capsule results in the preparation of approximately 5000 capsules having a dosage of 120 mg of terfenadine per capsule. One such capsule administered daily to a patient in need thereof provides symptomatic relief against histamine mediated allergies and other related conditions.

Obviously, the net amount of terfenadine per dosage unit can be either increased or decreased by increasing or decreasing either the amount of terfenadine in the hot melt composition within the limits disclosed, or by increasing or decreasing the total weight fill per capsule.

**Claims**

1. A hot melting sustained release pharmaceutical composition for filling capsules consisting of from 8 to 28 parts by weight of terfenadine or a pharmaceutically acceptable salt thereof, from 1 to 5 parts by weight of paraffin wax and from 68 to 92 parts by weight of polyethylene glycol.

2. A hot melt composition according to Claim 1 wherein the polyethylene glycol has an average molecular

4

weight of 3350.

3. A hot melt composition according to Claim 1 which comprises 1 part by weight of paraffin wax; 15 to 20 parts by weight of terfenadine or a pharmaceutically acceptable salt thereof; and from 75 to 85 parts by weight of polyethylene glycol having an average molecular weight of 3350.

4. A sustained release terfenadine composition in dosage unit form which comprises a hard or soft shelled capsule containing, in a hot melting composition from 60 to 180 mg of terfenadine or a pharmaceutically acceptable salt thereof, from 5 to 33 mg paraffin wax and from 450 to 610 mg of polyethylene glycol.

5. A terfenadine composition in dosage unit form according to Claim 4 comprising from 100 to 140 mg of terfenadine or a pharmaceutically acceptable salt thereof, from 5 to 10 mg of paraffin wax and from 500 to 580 mg of polyethylene glycol having an average molecular weight of 3350.

6. A terfenadine composition according to Claim 4 which is a hard shell terfenadine capsule consisting of 120 mg of terfenadine or a pharmaceutically acceptable salt thereof, 6.7 mg of paraffin wax and 543 mg of polyethylene glycol having an average molecular weight of 3350.

7. Process for preparing a terfenadine containing hot melt sustained release pharmaceutical composition for filling capsules, which comprises melting from 68 to 92 parts by weight of polyethylene glycol at a temperature from 60 to 80° C and then adding thereto from 8 to 28 parts by weight of terfenadine or a pharmaceutically acceptable salt thereof and from 1 to 5 parts by weight of paraffin wax under stirring.

8. A process as in Claim 7 wherein from 75 to 85 parts by weight of polyethylene glycol having an average weight of 3350, 15 to 20 parts by weight of terfenadine or a pharmaceutically acceptable salt thereof and 1 part by weight of paraffin wax are employed.

**Claims for the following Contracting State: AT**

1. A process for preparing a terfenadine containing hot melt sustained release pharmaceutical composition for filling capsules, which comprises melting from 68 to 92 parts by weight of polyethylene glycol at a temperature from 60 to 80° C and then adding thereto from 8 to 28 parts by weight of terfenadine or a pharmaceutically acceptable salt thereof and from 1 to 5 parts by weight of paraffin wax under stirring.

2. A process according to Claim 1 wherein the polyethylene glycol has an average molecular weight of 3350.

3. A process according to Claim 1 wherein from 75 to 85 parts by weight of polyethylene glycol having an average weight of 3350, 15 to 20 parts by weight of terfenadine or a pharmaceutically acceptable salt thereof and 1 part by weight of paraffin wax are employed.

4. A process according to Claim 1 for preparing a terfenadine composition in dosage unit form which comprises a hard or soft shelled capsule containing a hot melt composition comprising from 60 to 180 mg of terfenadine or a pharmaceutically acceptable salt thereof, from 5 to 33 mg paraffin wax and from 450 to 610 mg of polyethylene glycol.

5. A process according to Claim 1 for preparing a terfenadine composition in dosage unit form comprising from 100 to 140 mg of terfenadine or a pharmaceutically acceptable salt thereof, from 5 to 10 mg of paraffin wax and from 500 to 580 mg of polyethylene glycol having an average molecular weight of 3350.

6. A process according to Claim 1 for preparing a terfenadine composition which is a hard shell terfenadine capsule containing 120 mg of terfenadine or a pharmaceutically acceptable salt thereof, 6.7 mg of paraffin wax and 543 mg of polyethylene glycol having an average molecular weight of 3350.

**Revendications**

5

EP 0 173 293 B1

1. Une composition pharmaceutique à libération retardée fondant à la chaleur pour remplir des capsules, consistant en 8 à 28 parties en poids de terfénadine ou d'un de ses sels pharmaceutiquement acceptable, 1 à 5 parties en poids de cire de paraffine et 68 à 92 parties en poids de polyéthylèneglycol.

2. Une composition hot melt selon la revendication 1, dans laquelle le polyéthylèneglycol a un poids moléculaire moyen de 3 350.

3. Une composition hot melt selon la revendication 1, qui comprend : 1 partie en poids de cire de paraffine ; 15 à 20 parties en poids de terfénadine ou d'un de ses sels pharmaceutiquement acceptables ; et de 75 à 85 parties en poids de polyéthylèneglycol d'un poids moléculaire de 3 350.

4. Une composition de terfénadine à libération retardée sous forme de dose unitaire qui comprend une capsule à enveloppe dure ou molle contenant, dans une composition fondant à la chaleur, de 60 à 180 mg de terfénadine ou d'un de ses sels pharmaceutiquement acceptables, de 5 à 33 mg de cire de paraffine et de 450 à 610 mg de polyéthylèneglycol.

5. Une composition de terfénadine sous forme de dose unitaire selon la revendication 4, comprenant de 100 à 140 mg de terfénadine ou d'un de ses sels pharmaceutiquement acceptables, de 5 à 10 mg de cire de paraffine et de 500 à 580 mg de polyéthylèneglycol d'un poids moléculaire moyen de 3 350.

6. Une composition de terfénadine selon la revendication 4, qui est une capsule de terfénadine à enveloppe dure consistant en 120 mg de terfénadine ou d'un de ses sels pharmaceutiquement acceptables, 6,7 mg de cire de paraffine et 543 mg de polyéthylèneglycol d'un poids moléculaire moyen de 3 350.

7. Un procédé pour préparer une composition pharmaceutique hot melt de terfénadine à libération retardée pour le remplissage de capsules, qui consiste à faire fondre de 68 à 92 parties en poids de polyéthylèneglycol à une température de 60 à 80°C et ensuite à y ajouter en agitant de 8 à 28 parties en poids de terfé-nadine ou d'un de ses sels pharmaceutiquement acceptables et de 1 à 5 parties en poids de cire de paraffine.

8. Un procédé selon la revendication 7, dans lequel on utilise de 75 à 85 parties en poids de polyéthylèneglycol ayant un poids moléculaire moyen de 3 350, de 15 à 20 parties en poids de terfénadine ou d'un de ses sels pharmaceutiquement acceptable et 1 partie en poids de cire de paraffine.

**Revendications pour l'Etat contractant suivant: AT**

1. Un procédé pour préparer une composition pharmaceutique hot melt de terfénadine à libération retardée pour le remplissage de capsules, qui consiste à faire fondre de 68 à 92 parties en poids de polyéthylèneglycol à une température de 60 à 80°C et à y ajouter ensuite en agitant de 8 à 28 parties en poids de terfénadine ou d'un de ses sels pharmaceutiquement acceptables et de 1 à 5 parties en poids de cire de paraffine.

2. Un procédé selon la revendication 1, dans lequel le polyéthylèneglycol a un poids moléculaire moyen de 3 350.

3. Un procédé selon la revendication 1, dans lequel on utilise de 75 à 85 parties en poids de polyéthylèneglycol d'un poids moléculaire moyen de 3 350, de 15 à 20 parties en poids de terfénadine ou d'un de ses sels pharmaceutiquement acceptables et 1 partie en poids de cire de paraffine.

4. Un procédé selon la revendication 1 pour préparer une composition de terfénadine sous forme de dose unitaire qui comprend une capsule à enveloppe dure ou molle contenant une composition hot melt comprenant de 60 à 180 mg de terfénadine ou d'un de ses sels pharmaceutiquement acceptables, de 5 à 33 mg de cire de paraffine et de 450 à 610 mg de polyéthylèneglycol.

5. Un procédé selon la revendication 1 pour préparer une composition de terfénadine sous forme de dose

6

unitaire comprenant de 100 à 140 mg de terfénadine ou d'un de ses sels pharmaceutiquement acceptables, de 5 à 10 mg de cire de paraffine et de 500 à 580 mg de polyéthylèneglycol d'un poids moléculaire moyen de 3 350.

6. Un procédé selon la revendication 1 pour préparer une composition de terfénadine qui est une capsule de terfénadine à enveloppe dure contenant 120 mg de terfénadine ou d'un de ses sels pharmaceutiquement acceptables, 6,7 mg de cire de paraffine et 543 mg de polyéthylèneglycol d'un poids moléculaire moyen de 3 350.

## Patentansprüche

1. Pharmazeutische Heißschmelz-Zusammensetzung mit verzögerter Wirkstoffabgabe zum Füllen von Kapseln, bestehend aus 8 bis 28 Gewichtsteilen Terfenadine oder einem pharmazeutisch verträglichen Salz davon, 1 bis 5 Gewichtsteilen Paraffinwachs und 68 bis 92 Gewichtsteilen Polyethylenglykol.

2. Heißschmelz-Zusammensetzung nach Anspruch 1, wobei das Polyethylenglykol ein Molekulargewichtsmittel von 3350 aufweist.

3. Heißschmelz-Zusammensetzung nach Anspruch 1, die 1 Gewichtsteil Paraffinwachs; 15 bis 20 Gewichtsteile Terfenadine oder ein pharmazeutisch verträgliches Salz davon; und 75 bis 85 Gewichtsteile Polyethylenglykol mit einem Molekulargewichtsmittel von 3350 enthält.

4. Terfenadine-Zusammensetzung mit verzögerter Wirkstoffabgabe in Dosiseinheitsform, die eine hartschalige oder weichschalige Kapsel umfaßt, enthaltend in einer Heißschmelz-Zusammensetzung 60 bis 180 mg Terfenadine oder ein pharmazeutisch verträgliches Salz davon, 5 bis 33 mg Paraffinwachs und 450 bis 610 mg Polyethylenglykol.

5. Terfenadine-Zusmmensetzung in Dosiseinheitsform nach Anspruch 4, enthaltend 100 bis 140 mg Terfenadine oder ein pharmazeutisch verträgliches Salz davon, 5 bis 10 mg Paraffinwachs und 500 bis 580 mg Polyethylenglykol mit einem Molekulargewichtsmittel von 3350.

6. Terfenadine-Zusammensetzung nach Anspruch 4, bei der es sich um eine hartschalige Terfenadine-Kapsel handelt, bestehend aus 120 mg Terfenadine oder einem pharmazeutisch verträglichen Salz davon, 6,7 mg Paraffinwachs und 543 mg Polyethylenglykol mit einem Molekulargewichtsmittel von 3350.

7. Verfahren zur Herstellung einer Terfenadine enthaltenden pharmazeutischen Heißschmelz-Zusammensetzung mit verzögerter Wirkstoffabgabe zum Füllen von Kapseln, umfassend das Schmelzen von 68 bis 92 Gewichtsteilen Polyethylenglykol bei einer Temperatur von 60 bis 80° C und anschließend die Zugabe von 8 bis 28 Gewichtsteilen Terfenadine oder eines pharmazeutisch verträglichen Salzes davon und von 1 bis 5 Gewichtsteilen Paraffinwachs unter Rühren.

8. Verfahren nach Anspruch 7, wobei 75 bis 85 Gewichtsteile Polyethylenglykol mit einem Molekulargewichtsmittel von 3350, 15 bis 20 Gewichtsteile Terfenadine oder eines pharmazeutisch verträglichen Salzes davon und 1 Gewichtsteil Paraffinwachs verwendet werden.

## Patentansprüche für folgenden Vertragsstaat: AT

1. Verfahren zur Herstellung einer Terfenadine enthaltenden pharmazeutischen Heißschmelz-Zusammensetzung mit verzögerter Wirkstoffabgabe zum Füllen von Kapseln, umfassend das Schmelzen von 68 bis 92 Gewichtsteilen Polyethylenglykol bei einer Temperatur von 60 bis 80° C und anschließend die Zugabe von 8 bis 28 Gewichtsteilen Terfenadine oder eines pharmazeutisch verträglichen Salzes davon und von 1 bis 5 Gewichtsteilen Paraffinwachs unter Rühren.

2. Verfahren nach Anspruch 1, wobei das Polyethylenglykol ein Molekulargewichtsmittel von 3350 aufweist.

3. Verfahren nach Anspruch 1, wobei 75 bis 85 Gewichtsteile Polyethylenglykol mit einem Molekulargewichtsmittel von 3350, 15 bis 20 Gewichtsteile Terfenadine oder eines pharmazeutisch verträglichen Salzes davon und 1 Gewichtsteil Paraffinwachs verwendet werden.

4. Verfahren nach Anspruch 1 zur Herstellung einer Terfenadine-Zusammensetzung in Dosiseinheitsform, die eine hartschalige oder weichschalige Kapsel mit einer Heißschmelz-Zusammensetzung umfaßt, enthaltend 60 bis 180 mg Terfenadine oder ein pharmazeutisch verträgliches Salz davon, 5 bis 33 mg Paraffinwachs und 450 bis 610 mg Polyethylenglykol.

5. Verfahren nach Anspruch 1 zur Herstellung einer Terfenadine-Zusammensetzung in Dosiseinheitsform, umfassend 100 bis 140 mg Terfenadine oder eines pharmazeutisch verträglichen Salzes davon, 5 bis 10 mg Paraffinwachs und 500 bis 580 mg Polyethylenglykol mit einem Molekulargewichtsmittel von 3350.

6. Verfahren nach Anspruch 1, zur Herstellung einer Terfenadine-Zusammensetzung, bei der es sich um eine hartschalige Terfenadine-Kapsel mit einem Gehalt an 120 mg Terfenadine oder eines pharmazeutisch verträglichen Salzes davon, 6,7 mg Paraffinwachs und 543 mg Polyethylenglykol mit einem Molekulargewichtsmittel von 3350 handelt.